# EUROPÄISCHE PATENTANMELDUNG

(11) **EP 1 318 130 A1**
(43) Veröffentlichungstag der Anmeldung: **11.06.2003**
(21) Anmeldenummer: 02026362.0
(22) Anmeldetag: 25.11.2002
(51) Int. Cl.: C07C 29/145, C07C 35/08

(54) **Verfahren zur Herstellung von 3,3,5-Trimethylcyclohexanol**

(30) Priorität: 06.12.2001 DE 10160009
(71) Anmelder: HAARMANN & REIMER GMBH, 37603 Holzminden (DE)
(72) Erfinder: Kuhn, Walter, Dr,, 37603 Holzminden (DE); Funk, Hans-Ulrich, 37697 Lauenförde (DE); Senft, Gerhard, 37603 Holzminden (DE); Quest, Heinz-Dieter, 37671 Höxter (DE)
(74) Vertreter: Eisenführ, Speiser & Partner

(57) **Zusammenfassung**

Die Erfindung betrifft ein Verfahren zur Herstellung von 3,3,5-Trimethylcyclohexanol durch Hydrierung von Isophoron in Gegenwart eines Rutheniumkatalysators.

## Beschreibung

Die Erfindung betrifft ein Verfahren zur Herstellung von 3,3,5-Trimethylcyclohexanol durch Hydrierung von Isophoron (3,3,5-Trimethyl-2-cyclohexen-1-on) in Gegenwart eines Rutheniumkatalysators.

3,3,5-Trimethylcyclohexanol ist ein Geschmackstoff mit mentholartigem Charakter. (S. Arctander, Perfume and Flavour Chemicals, No. 2998, 1969 Montclair, N.J. USA).

Des weiteren kann 3,3,5-Trimethylcyclohexanol als Ausgangsmaterial für die Herstellung von Homomenthylsalicylat (3,3,5-Trimethylcyclohexylsalicylat) eingesetzt werden, welches als UV-Filter Verwendung findet.

In J. Amer. Pharm. Assoc. 1942, 25 wird Isophoron mittels Platin zu 3,3,5-Trimethylcyclohexanol bei Raumtemperatur hydriert. Der Katalysator wird im Verhältnis 1 : 17,5 zu Isophoron eingesetzt.

In Chem. and Ind. 1933, 518 ist die Hydrierung von Isophoron unter Raney-Nickel-Katalyse oder Kupferchromit-Katalyse erwähnt. Es entsteht ein Gemisch aus 3,3,5-Trimethylcyclohexanol und 3,3,5-Trimethylcyclohexanon.

In Neftepererab. Neftekhim. (Moscow) 1971, 5, 41 ist die Hydrierung von Isophoron zu 3,3,5-Trimethylcyclohexanol unter Raney-Nickel-Katalyse bei 15 bis 140°C beschrieben. Die Hydrierung wird bei einem Druck von 35-100 bar durchgeführt und ergibt 3,3,5-Trimethylcyclohexanol mit 90 % Ausbeute.

Nach den beschriebenen Verfahren lässt sich 3,3,5-Trimethylcyclohexanol nur mit unbefriedigender Ausbeute und/oder unter Verwendung größerer Mengen an Katalysatoren herstellen, die zudem teilweise cancerogen sind.

Es bestand daher die Aufgabe, ein Verfahren zur Herstellung von 3,3,5-Trimethylcyclohexanol zu finden, welches 3,3,5-Trimethylcyclohexanol in guter Ausbeute und in hoher Reinheit liefern kann.

Die vorliegende Erfindung betrifft daher ein Verfahren zur Herstellung von 3,3,5-Trimethylcyclohexanol durch Hydrierung von Isophoron, dadurch gekennzeichnet, dass die Hydrierung in Gegenwart eines Katalysators enthaltend Ruthenium durchgeführt wird.

Das erfindungsgemäße Verfahren ermöglicht insbesondere die Herstellung von sensorisch einwandfreiem 3,3,5-Trimethylcyclohexanol unter wirtschaftlichen Aspekten, auch im industriellen Maßstab.

Das erfindungsgemäß hergestellte 3,3,5-Trimethylcyclohexanol kann beispielsweise als Geschmackstoff und zur Herstellung von Homomenthylsalicylat, einem gängigen UV-Filter, eingesetzt werden.

Das erfindungsgemäße Verfahren kann durch die folgende Reaktionsgleichung dargestellt werden:

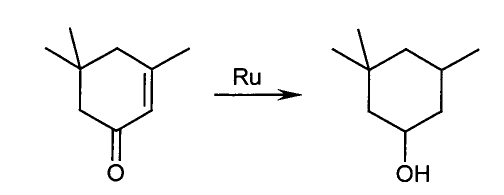

Für das erfindungsgemäße Verfahren kann der Katalysator im trockenen oder feuchten Zustand (Wassergehalt bis zu 60 Gew.-%) verwendet werden.

Die erfindungsgemäßen Katalysatoren enthalten Ruthenium in elementarer, metallischer Form.

Das Ruthenium kann beispielsweise in fein verteilter Form, aufgebracht auf Träger oder zusammen mit anderen Metallen eingesetzt werden (z.B. Mischungen, Legierungen). Die Katalysatoren können Dotierungen mit einem oder mehreren Metallen enthalten.

Das Ruthenium kann auf organische oder anorganische Trägermaterialien aufgebracht sein. Die Katalysatoren können ein Trägermaterial oder Mischungen von Trägermaterialien enthalten. Als vorteilhafte Trägermaterialien seien genannt: Aktivkohle, Kohle, Aluminiumoxide, Metalloxide, Kieselgele, Zeolithe, Tone, Tongranulate, amorphe Aluminiumsilicate oder sonstige anorganische Träger. Bevorzugte Trägermaterialien sind Aktivkohle, Siliciumdioxid, Calciumcarbonat und Aluminiumoxid. Besonders bevorzugtes Trägermaterial ist Aktivkohle.

Ein ganz besonders bevorzugter Katalysator ist Ruthenium auf Aktivkohle.

Die Katalysatoren können als Formkörper wie beispielsweise Hohlsträngen, Tabletten, Extrudaten, Kugeln, Zylindern, Würfeln, Kegeln und dergleichen eingesetzt werden, was besonders bei einer kontinuierlichen Reaktionsführung vorteilhaft ist.

Werden Trägermaterialien enthaltende Katalysatoren verwendet, kann der Anteil an Ruthenium auf dem Trägermaterial im allgemeinen 0,5 bis 50 Gew.-%, bevorzugt 1 bis 20 Gew.-%, insbesondere bevorzugt 3 bis 10 Gew.-%, bezogen auf den trockenen Katalysator, betragen.

Es ist ebenfalls möglich das Ruthenium-Metall vor Beginn oder während der Hydrierung in situ durch Reduktion mit Wasserstoff aus entsprechenden Verbindungen wie Oxiden oder Salzen zu erzeugen, wobei gegebenenfalls das Ruthenium auf einen Träger niedergeschlagen werden kann. Geeignet hierfür sind beispielsweise Rutheniumtetroxid, Rutheniumtetroxid-hydrat oder auch Rutheniumhalogenide.

Für das erfindungsgemäße Verfahren liegt das Gewichtsverhältnis von Isophoron zu Ruthenium-Metall im Bereich 500.000 bis 100 : 1, vorteilhaft im Bereich 200.000 bis 200 : 1, bevorzugt im Bereich 50.000 bis 500 : 1, insbesondere bevorzugt im Bereich 30.000 bis 5.000 : 1.

Die Ruthenium-Metallmenge bezieht sich dabei auf den absoluten Gehalt des Rutheniums, d.h. ohne Trägermaterial und ohne eventuell vorhandenes Wasser oder andere Bestandteile des Katalysators.

Das Verfahren wird erfindungsgemäß bei 30 bis 220°C, bevorzugt bei 60 bis 190°C und insbesondere bevorzugt bei 90 bis 160°C durchgeführt.

Das erfindungsgemäße Verfahren wird mit Wasserstoff durchgeführt, die Wasserstoffdrücke liegen üblicherweise im Bereich 1 bis 100 bar abs., bevorzugt ist eine Reaktionsführung bei Wasserstoffdrücken im Bereich 5 bis 50 bar abs., insbesondere im Bereich 10 bis 20 bar abs..

Die Hydrierungszeit liegt üblicherweise im Bereich 2 bis 100 Stunden, bevorzugt im Bereich 5 bis 40 Stunden.

Das Verfahren kann kontinuierlich, semi-kontinuierlich und diskontinuierlich durchgeführt werden.

Das erfindungsgemäße Verfahren kann unter Verwendung von Lösungsmitteln oder Lösungsmittelgemischen durchgeführt werden. Geeignet sind beispielsweise Alkohole, wässrige Alkohole, Ether, Ester, aromatische oder gesättigte Kohlenwasserstoffe. Beispielsweise können Lösungsmittel wie Methanol, Ethanol, Isopropanol, n-Propanol, Isobutanol, n-Butanol, sek.-Butanol, Tetrahydrofuran, Dibutylether, Ethylenglykoldimethylether, Ethylacetat, Methylacetat, Pentan, Hexan, Heptan, Octan, Cyclopentan, Cyclohexan, Methylcyclohexan, Cyclooctan, Benzol, Toluol, Ethylbenzol oder Xylole verwendet werden.

Bevorzugt wird das Verfahren lösungsmittelfrei durchgeführt.

Das erfindungsgemäße Verfahren kann beispielsweise folgendermaßen durchgeführt werden:

In einem Druckbehälter werden Isophoron und der Katalysator vorgelegt. Es wird bei der gewählten Temperatur und dem gewähltem Wasserstoffdruck hydriert. Nach Ende der Hydrierung kann das rohe 3,3,5-Trimethylcyclohexanol durch Entfernen des Katalysators (z.B. Filtration, Dekantierung, Zentrifugierung) erhalten werden. Bei Bedarf kann eine weitere Reinigung des 3,3,5-Trimethylcyclohexanols erfolgen, beispielsweise durch Destillation.

Mit dem erfindungsgemäßen Verfahren können, je nach Reaktionsbedingungen, unterschiedliche Verhältnisse an cis- und trans-Isomeren im Hydrierungsprodukt erzielt werden. Typischerweise liegt das Verhältnis von cis- : trans-Isomer im Bereich von etwa 60 : 40 bis 90 : 10. Insbesondere bei längeren Hydrierzeiten entsteht 3,3,5-Trimethylcyclohexanol mit einem hohen Anteil an cis-Isomer. Ein 57 : 43 - Isomerengemisch (cis:trans) weist beispielsweise im Unterschied zum bekannten und auch marktüblichen 90 : 10 - Isomerengemisch einen frischeren Geschmack auf.

Das folgende Beispiel erläutert die Erfindung:

### Beispiel

In einen 5 l Rührautoklaven mit Begasungsrührer wurden 2493 g Isophoron und 5,8 g Ruthenium auf Aktivkohle (Ru-Gehalt: 5 Gew.-%, Wassergehalt: ca. 55 Gew.-%; das Verhältnis von Ru zu Isophoron lag demnach bei etwa 1 : 19.000) vorgelegt. Es wurde 7,5 Stunden bei 140°C und 18 bar Wasserstoffdruck hydriert. Nach der Hydrierung wurden 2553 g 3,3,5-Trimethylcyclohexanol mit einer GC-Reinheit von 99,7 Gew.-% erhalten. Das erhaltene 3,3,5-Trimethylcyclohexanol kann bei Bedarf weitestgehend ohne Rückstand bei 130°C Sumpftemperatur und 10 mbar destilliert werden. Das cis-: trans-Isomerenverhältnis lag bei 92 : 8.

## Patentansprüche

1. Verfahren zur Herstellung von 3,3,5-Trimethylcyclohexanol durch Hydrierung von Isophoron, **dadurch gekennzeichnet, dass** die Hydrierung in Gegenwart eines Katalysators enthaltend Ruthenium durchgeführt wird.

2. Verfahren nach Anspruch 1, **dadurch gekennzeichnet, dass** als Katalysator Ruthenium auf Aktivkohle verwendet wird.

3. Verfahren nach Anspruch 1 oder 2, **dadurch gekennzeichnet, dass** das Gewichtsverhältnis von Isophoron zu Ruthenium-Metall im Bereich 500.000 bis 100 : 1 liegt.

4. Verfahren nach mindestens einem der Ansprüche 1 bis 3, **dadurch gekennzeichnet, dass** die Umsetzung bei einer Temperatur im Bereich von 30 bis 220°C durchgeführt wird.

5. Verfahren nach mindestens einem der Ansprüche 1 bis 4, **dadurch gekennzeichnet, dass** die Hydrierung bei einem Wasserstoffdruck von 1 bis 100 bar abs. durchgeführt wird.

6. Verfahren nach mindestens einem der Ansprüche 1 bis 5, **dadurch gekennzeichnet, dass** die Hydrierung lösungsmittelfrei durchgeführt wird.
